# EUROPÄISCHE PATENTANMELDUNG

(11) **EP 3 988 135 A1**
(43) Veröffentlichungstag der Anmeldung: **27.04.2022**
(21) Anmeldenummer: 21201793.3
(22) Anmeldetag: 08.10.2021
(51) Int. Cl.: A61L 9/14

(54) **VERFAHREN UND VORRICHTUNG ZUR REINIGUNG VON VIRIONENENBELASTETER LUFT, SOWIE VERWENDUNG DAZU**

(30) Priorität: 08.10.2020 DE 102020126375
(71) Anmelder: Barnstedt, Iris, 77749 Hohberg (DE)
(72) Erfinder: Barnstedt, Iris, 77749 Hohberg (DE)
(74) Vertreter: Tahhan, Nader Isam Mark

(57) **Zusammenfassung**

Die Erfindung betrifft ein Verfahren und eine Vorrichtung zur Reinigung von mit behüllten Virionen belasteter Luft.

Das Verfahren dient der Reduktion der Last behüllter Viren in aerosolhaltiger Umgebungsluft, wobei die Umgebungsluft mit einer Reinigungsflüssigkeit (F) in Kontakt gelangt. Es ist dadurch gekennzeichnet, dass die Reduktion durch eine Inaktivierung der in den Aerosolen enthaltenen behüllten Viren erreicht wird, und die Reinigungsflüssigkeit (F) tensidhaltig ist. Die Inaktivierung der Viren erfolgt durch Interaktion in den Aerosolen mittels Gelangens des unbeeinflussten, fettlösenden Tensids aus der Reinigungsflüssigkeit (F) in die Aerosole und der Abtransport der inaktivierten Virenreste erfolgt mittels der Aerosole in die Umgebungsluft.

Die Vorrichtung umfasst ein Gehäuse (1) und ein Gebläse (3), sowie eine Einrichtung zur Bereitstellung einer mit einer Reinigungsflüssigkeit (F) benetzbaren Oberfläche, und ist dadurch gekennzeichnet, dass die Reinigungsflüssigkeit (F) tensidhaltig und fettlösend ist.

## Beschreibung

### Einleitung

Die Erfindung betrifft ein Verfahren und eine Vorrichtung zur Reinigung von Luft und anderen Gasen. Insbesondere betrifft die Erfindung ein Verfahren und eine Vorrichtung zur Reinigung von mit behüllten Virionen belasteter Luft, sowie die Verwendung der Vorrichtung unter Nutzung des Verfahrens.

### Stand der Technik und Nachteile

Die gesundheitsschädliche Wirkung von Viren auf Menschen ist hinlänglich bekannt. Typische Infektionswege sind die Tröpfcheninfektion, beispielsweise durch Anhusten oder Anniesen; Schmierinfektionen aufgrund der Berührung kontaminierter Oberflächen oder direkten Kontakt zu infizierten Personen; und die Infektion aufgrund von Aerosolen, also belasteter Atemluft. Letzterer Infektionsweg ist - je nach Virus - für bis zu 50% der Infektionen verantwortlich. Insbesondere in geschlossenen Räumen steigt die Infektionsgefahr durch Aerosole weiter an. Dementsprechend ist es wünschenswert, die Belastung der Atemluft mit Virionen, also außerhalb der Zelle befindlichen Viren, in Innenräumen soweit als möglich zu reduzieren.

Hierfür sind aus dem Stand der Technik verschiedenartige Lösungen bekannt.

Im einfachsten Fall wird durch intensives Lüften für einen großzügigen Luftaustausch gesorgt. Dies ist jedoch nicht für alle Räume möglich, da nicht jeder Raum einen entsprechenden Zugang zur Außenluft aufweist. Zudem erzeugt das Lüften häufig einen schnell als unangenehm empfundenen Luftzug. Auch der Energieverlust bei niedrigen oder auch hohen Außentemperaturen aufgrund verlorener Heiz- oder Kühlenergie ist nachteilig.

Bekannt sind Vorrichtungen zur Reinigung von (Atem-)Luft mittels Schwebstofffiltern, sogenannten HEPA-Filtern, welche Aerosole und die darin befindlichen Virionen herausfiltern, also zurückhalten. Der Schwebstofffilter befindet sich in einem Luftstrom, welcher durch einen Ventilator in Bewegung gesetzt wird. Wenn sowohl die angesogene, belastete, als auch die ausgestoßene, gereinigte Luft im Raum verbleibt (Umwälzung), geht keine Heiz- bzw. Kühlenergie verloren. Bei ausreichender Dimensionierung kann die Luft auch so schnell ausgetauscht werden, dass die verbleibende Virionenlast sehr gering ist. Nachteilig an dieser Lösung ist jedoch die Notwendigkeit der Wartung der Vorrichtung durch Reinigen oder Austausch des Schwebstofffilters, was zu entsprechenden Kosten und Stillstandszeiten führt. Ein weiterer Nachteil liegt in der Notwendigkeit des nach geltenden Regeln erforderlichen Integritätstests einer solchen Anlage nach dem Einbau eines neuen oder gereinigten Filters. Dies ist nur durch speziell eingewiesenes Personal möglich. Hinzu kommt das Entstehen von großen Abfallmengen oder gar kontaminiertem Sondermüll, aufgrund des Austauschs der genutzten Filter gegen frische. Eine derartige Reinigungsvorrichtung ist beispielsweise aus der Druckschrift EP 1 600 201 A1 bekannt.

Genutzt wird weiter die Behandlung der Atemluft mittels ultraviolettem Licht, dessen Virionen reduzierende Wirkung ebenfalls seit langem bekannt ist. Auch hier wird vorzugsweise Luft mittels eines Gebläses durch ein Gehäuse gefördert. Dieses ist von außen lichtdicht, da die UV-Strahlung selber gesundheitsschädlich ist. Nachteilig ist auch hier die Notwendigkeit der Wartung aufgrund begrenzter Lebensdauer der UV-Lichtquelle, sowie der nicht unerhebliche Energiebedarf zum Betrieb derselben. Weiterhin lassen sich die Lichtquellen nach heutigem Stand der Technik nur als Quecksilberlampen herstellen und sind somit schwermetallbelastet. Ein Beispiel für eine Luftdesinfektion mittels UV-Licht findet sich in Druckschrift US 5,656,242. Hier wird außerdem noch Ozon generiert, welches zwar für Mikroorganismen ein Biozid darstellt, jedoch in Atemluft unerwünscht ist.

Andere Vorrichtungen zur Desinfektion von Raumluft nutzen chemische Wirkstoffe wie Ethanol, Formaldehyd, oder Wasserstoff-Peroxid. Derartige Stoffe sind zwar wirkungsvoll, haben jedoch Nachteile wie eine mögliche Explosionsgefahr, oder sind aufgrund von Beschränkungen im sog. MAK-Wert (Maximale Arbeitsplatzkonzentration) nur eingeschränkt oder überhaupt nicht für Innenräume zur Luftumwälzung, sondern nur zur Abgabe in die Außenluft verwendbar.

### Aufgabe der Erfindung und Lösung

Der Erfindung liegt demnach die Aufgabe zugrunde, ein Verfahren und eine Vorrichtung bereitzustellen, welches bzw. welche die Nachteile des Standes der Technik vermeidet.

Aufgrund der zunehmenden Relevanz der Elimination von behüllten Virionen, nachfolgend vereinfachend auch "Viren" genannt, soll die Erfindung insbesondere der Reinigung von derartigen Viren dienen.

Die Erfindung soll für jegliche Räume, also auch solche ohne direkten Zugang zu Frischluft, einsetzbar sein. Die Erfindung ist zur Reinigung von Raum- und Umgebungsluft vorgesehen und dazu geeignet, entsprechende Mengen in Abhängigkeit von Raumvolumina und Luftwechselrate (z.B. mit 6-fachem Luftwechsel 800m³/h für ein Raumvolumen von 133 m³) zu behandeln.

Der Energieverlust bei Verwendung der Vorrichtung soll möglichst gering sein.

Die Vorrichtung soll möglichst unterbrechungs- und wartungsfrei betreibbar sein, bzw. notwendige Wartung soll auch durch unerfahrenes Personal möglich sein.

Eine gesundheitsschädigende Wirkung durch den Betrieb der Vorrichtung soll ausgeschlossen sein.

Die Belastung der Umwelt aufgrund des Betriebs der Vorrichtung soll minimal sein.

Die Aufgabe wird durch ein Verfahren nach Anspruch 1, eine Filterstufe nach nebengeordnetem Anspruch 7, eine Vorrichtung nach nebengeordnetem Anspruch 10, sowie eine Verwendung nach Anspruch 13 gelöst. Vorteilhafte Ausführungsformen sind den jeweils abhängigen Unteransprüchen, der nachfolgenden Beschreibung sowie den Figuren zu entnehmen.

### Beschreibung

Zunächst erfolgt eine Beschreibung des erfindungsgemäßen Verfahrens. Daran schließt sich die Beschreibung der erfindungsgemäßen Filterstufe und Vorrichtung, sowie einer Verwendung derselben an.

Die Erfindung betrifft ein Verfahren zur Reduktion der Last behüllter Viren (genauer: Virionen) in aerosolhaltiger Umgebungsluft. Derartige Viren werden zunehmend relevant, weswegen ein besonderes Interesse an einer Elimination gerade derartiger Viren aus der Atemluft besteht. Bekanntermaßen heften sich Viren an kleinste Flüssigkeitspartikel (Aerosole), die beim Ausatmen infizierter Personen in die Umgebungsluft gelangen. Beim Einatmen von mit derartigen Viren belasteter Luft kann sich eine einatmende Person ihrerseits infizieren. In geschlossenen Räumen, und insbesondere in solchen mit schlechten Lüftungsmöglichkeiten, ist eine laufende Reduktion der Last behüllter Viren in den Aerosolen von großer Wichtigkeit.

Es sei angemerkt, dass auch dann, wenn nachfolgend verkürzt von "Viren" gesprochen wird, immer behüllte Viren bzw. Virionen gemeint sind.

Zur Reduktion der Virenlast wird die Umgebungsluft mittels eines Gebläses durch ein Gehäuse gefördert. In diesem Gehäuse gelangt sie, und mit ihr die kontaminierten Aerosole, mit einer "Reinigungsflüssigkeit" in Kontakt. Dieser Kontakt resultiert in einer Reduktion der in der Umgebungsluft befindlichen Last behüllter Viren. Es sei angemerkt, dass der Begriff der "Reinigung" allgemein zu verstehen ist, und keine Einschränkung hinsichtlich eines möglichen Entfernens der Viren aus den Aerosolen bedeutet. Insofern meint der Begriff "Reinigungsflüssigkeit" lediglich, dass die Last behüllter Virionen in der Umgebungsluft reduziert, und diese somit "gereinigt" wird.

Die Reduktion wird durch eine Inaktivierung der mit der Reinigungsflüssigkeit in Kontakt gelangenden, in den Aerosolen enthaltenen behüllten Viren erreicht. Im Gegensatz zu den aus dem Stand der Technik bekannten Verfahren erfolgt demnach weder eine Filterung mittels mechanischer Mittel, also ein (möglichst) vollständiges Entfernern der Viren aus der Luft, was die o.g. Nachteile (Filterwechsel, Kosten, Stillstandszeiten) mit sich bringt. Auch findet keine Desinfektion der Luft statt, also kein (möglichst) vollständiges Abtöten jeglicher, auch ggf. nicht gesundheitsschädlicher, Organismen, verbunden mit den Umweltbelastungen, die mit einer Desinfektion einhergehen (hoher Energieverbrauch oder gesundheitsschädliche Desinfektionsmittel).

Stattdessen werden die in den Aerosolen enthaltenden Viren lediglich inaktiviert, was ohne mechanische Filter, den Einsatz von energieintensiver Strahlung, oder gesundheitsbedenkliche Chemikalien möglich ist.

Die Reduktion wird konkret dadurch erreicht, dass die Reinigungsflüssigkeit tensidhaltig ist. Versuche haben ergeben, dass eine tensidhaltige Reinigungsflüssigkeit zu einer signifikanten Reduzierung der Belastung von mit behüllten Viren belasteten Aerosolen in Umgebungsluft erreichbar ist. Die lipidhaltige Virushülle wird durch das Tensid, welches vorliegend vom fettlösenden (und nicht vom eiweißlösenden) Typ ist, zerstört bzw. aufgebrochen und das Virus somit inaktiviert. Es sei angemerkt, dass ein nicht mehr infektiöser, inaktivierter Rest des Virions durchaus im Luftstrom und/oder in der Reinigungsflüssigkeit verbleiben kann, ohne dass gesundheitliche Folgen befürchtet werden müssen.

Der Anteil des Tensids an der Reinigungsflüssigkeit beträgt typischerweise 0,1% bis 99,9%. Vorzugsweise liegt er jedoch im Bereich 0,25% bis 90%, weiter bevorzugt zwischen 0,4% bis 20%, und besonders bevorzugt im Bereich 0,5% bis 2%.

Als Wirkstoffe sind grundsätzlich alle bekannten Tenside vom fettlösenden Typ verwendbar. Insbesondere sind die Tenside möglichst biologisch abbaubar und damit umweltschonend. Die Hauptmerkmale werden hierbei auf die lipidlösende Eigenschaft gelegt. Demgegenüber kommen für das erfindungsgemäße Verfahren eiweißlösende Flüssigkeiten nicht in Betracht, da diese nicht die Virushülle, sondern den Proteine enthaltenden Kern zerstören. Auch soll die vollständige Benetzbarkeit von Oberflächen gewährleistet sein, sodass eine kontinuierliche Phasentrennschicht zwischen Flüssigkeit und Gasphase entsteht. Sowohl nichtionische, als auch anionische oder kationische Tenside kommen hier in Frage. Die Reinigungsflüssigkeit entsteht durch das Mischen von Wasser und Tensid zu einem Detergent, wobei eine Schaumbildung zu vermeiden ist.

Erfindungsgemäß erfolgt die Reduktion der Belastung (zumindest ganz vorwiegend) mittels Interaktion in den Aerosolen, und Abtransport mittels derselben. Mit anderen Worten, beim Kontakt zwischen einer Grenzfläche der Reinigungsflüssigkeit und dem Aerosol gelangt Tensid in das Aerosol, wo es - ggf. nach Wiederablösen des Aerosols von der Reinigungsflüssigkeit - zur Inaktivierung der Virionen durch Auflösung ihrer Hülle führt. Es wird demnach lediglich die lipidhaltige Virushülle zerstört, und das Virus somit inaktiviert. Die proteinhaltigen Bestandteile des Virions werden erfindungsgemäß nicht in der oder durch die Reinigungsflüssigkeit aufgelöst, sondern bleiben intakt; sie sind jedoch nicht mehr gesundheitsschädlich. Der Abtransport dieser Viren(-reste) erfolgt dann vorzugsweise mittels der Aerosole. Da diese nur noch inaktive Viren(-reste) enthalten, sind auch sie nicht mehr gesundheitsschädlich. Da das Tensid über die Grenzfläche der Reinigung in die Aerosole gelangt, bedeutet dies auch, dass die Viren nicht, wie bei bekannten Verfahren, in Lösung mit der Reinigungsflüssigkeit gehen, wo sie zwecks ihrer Zerstörung einer chemischen und/oder physikalischen Behandlung unterzogen würden.

Dabei wird das Tensid bzw. die es enthaltende Reinigungsflüssigkeit unbeeinflusst gelassen. Das bedeutet, dass die Reinigungsflüssigkeit weder erwärmt, noch anderweitig modifiziert wird. Insbesondere erfolgt auch kein notwendiger "Aktivierungsschritt", in welchem die Reinigungsflüssigkeit oder ihre die Virenhüllen auflösenden Bestandteile zunächst "aktiviert" werden müssten.

Die Erfindung vermeidet somit die aus dem Stand der Technik bekannten Nachteile.

Die Erfindung dient explizit der Elimination von behüllten Viren aus der Umgebungsluft. Insofern wirkt die Erfindung auch selektiv, im Gegensatz zu einem Schwebstofffilter. Der Energieverlust bei Verwendung der Vorrichtung ist sehr gering, da lediglich ein Gebläse sowie eine Pumpe zum Betrieb benötigt werden. Das Verfahren kann kontinuierlich betrieben werden, ohne dass Luftmengen durch erhöhte Druckverluste o.ä. Einflussfaktoren (Filterbeladung, Herabsetzung von UV-Leistungen) vermindert würden. Zudem ist eine Wartung auch durch unerfahrenes Personal möglich. Bei der Durchführung des Verfahrens tritt auch keine gesundheitsschädigende Wirkung ein. Schließlich fallen bei der Durchführung keine umweltbelastenden Stoffe wie belastete Schwebstofffilter oder verbrauchte, schwermetallhaltige Quecksilberdampflampen an. Im Gegensatz zu Verfahren, bei welchen die proteinhaltigen Teile der Viren zerstört werden, was den Einsatz von gesundheitsschädlichen Chemikalien oder energieintensiven Beleuchtungen mit sich bringt, basiert das erfindungsgemäße Verfahren auf der Inaktivierung der Viren mittels Zerstörung ihrer Hülle; demnach ist es auch ausschließlich bei behüllten Viren einsetzbar.

Nachfolgend werden verschiedene Ausführungsformen des Verfahrens näher beschrieben.

Nach einer bevorzugten Ausführungsform wird die zu reinigende Umgebungsluft an einer einen Tensidfilm bereitstellenden Einrichtung zur Bereitstellung einer mit einer Reinigungsflüssigkeit benetzten Oberfläche wie einer Füllkörperkolonne oder vergleichbaren oberflächvergrößernden Geometrien entlang geführt. Im Folgenden wird vereinfachend von Füllkörperkolonnen gesprochen, welche selbstverständlich durch Festpackungen oder ähnlich wirkende Komponenten ersetzt werden können. Die Füllkörperkolonne bzw. gleichwirkende Komponente ist derart ausgelegt, dass die Umgebungsluft eine Grenzfläche des Tensidfilmes passieren muss. Dabei ist die Kontaktzeit zwischen Aerosolen und Grenzfläche dergestalt ausgewählt, dass das Tensid die in den Aerosolen enthaltenen Lipidhüllen der behüllten Viren aufbricht, wodurch diese behüllten Viren inaktiviert werden.

Anders ausgedrückt, das Gebläse transportiert einen Strom aerosolhaltiger Luft an der Füllkörperkolonne entlang. Diese wird mit Reinigungsflüssigkeit benetzt. Die Oberfläche der Füllkörperkolonne ist möglichst groß, so dass ein möglichst guter Kontakt zwischen aerosolhaltiger Luft und Reinigungsflüssigkeit ermöglicht wird. Beim Kontakt wirkt das Tensid dann auf die Virushüllen ein und schädigt diese. Die so inaktivierten Viren(-reste) können mittels der Aerosole abtransportiert werden. Sollten sie in geringem Umfang in die Reinigungsflüssigkeit übergehen, können sie dort bis zu einer Entsorgung der Flüssigkeit verbleiben. Selbst ein Körperkontakt mit der Flüssigkeit ist jederzeit unbedenklich, da von den Virenresten keinerlei Gefahr ausgeht.

Die Flüssigkeit kann dann bei Bedarf erneuert werden. Dies kann durch Entsorgen der Flüssigkeit, und/oder durch Hinzufügen von frischen Tensiden erreicht werden. Somit ist die erfindungsgemäße Lösung sehr umweltfreundlich, und schließt die Gefahr der Kontamination von Wartungspersonal faktisch aus.

Nach einer weiteren Ausführungsform wird die Umgebungsluft im Wege des Kontaktierens der Reinigungsflüssigkeit befeuchtet, um die Lebensdauer von Viren in der Umgebungsluft zu reduzieren. Anders ausgedrückt, die Anhebung der Luftfeuchtigkeit im Inneren einer das erfindungsgemäße Verfahren durchführenden Vorrichtung führt zu einer Verringerung der Lebensfähigkeit der in den Aerosolen enthaltenen behüllten Viren. Demnach ist die Belastung der mit dem erfindungsgemäßen Verfahren behandelten Luft weiter verringert.

Besonders bevorzugt wird die aus dem Gehäuse herausgeförderte, gereinigte, einem Innenraum entstammende Umgebungsluft in einen, und bevorzugt diesen, Innenraum auch wieder zurückgefördert. Demnach fließt die Umgebungsluft in einem Kreislauf, so dass sich Ihre Temperatur beim Reinigen nur unwesentlich ändert. Demnach kann das erfindungsgemäße Verfahren sehr energieeffizient durchgeführt werden; ein Aufheizen oder Abkühlen (Klimatisieren) kälterer oder wärmerer Außenluft entfällt. Selbstverständlich kann das Verfahren jedoch mit klimaverbessernden Behandlungen der Umgebungsluft ergänzt oder kombiniert werden; insbesondere die vorstehend genannte Befeuchtung kann hierzu gezählt werden.

Nach einer Ausführungsform fließt die Reinigungsflüssigkeit mittels Schwerkraft entlang der Einrichtung zur Bereitstellung einer mit einer Reinigungsflüssigkeit benetzten Oberfläche und sammelt sich in einem unterhalb derselben angeordneten Sammelbehälter. Aus diesem wird sie - ggf. in Abhängigkeit des pH-Wertes - erneut auf die Einrichtung gegeben, oder als unbedenkliches Schmutzwasser entsorgt.

Die mittels Schwerkraft bewirkte Strömung der Reinigungsflüssigkeit entlang der Einrichtung ist sehr energieeffizient und zudem geräuscharm. Zum Transportieren der Flüssigkeit an das obere Ende der Einrichtung genügt eine kleine Flüssigkeitspumpe.

Der Sammelbehälter dient dem Auffangen der herabrieselnden Flüssigkeit; bevorzugt kann hier auch ein pH-Wert-Sensor angeordnet sein. Auf die vorstehenden Erläuterungen zum pH-Wert wird verwiesen.

Nach einer besonders bevorzugten Ausführungsform ist das Tensid biologisch abbaubar. Insbesondere ist es nach gemäß Verordnung (EG) Nr. 648/2004 bzw. entsprechend dem Gesetz über die Umweltverträglichkeit von Wasch- und Reinigungsmitteln (WRMG, Wasch- und Reinigungsmittelgesetz in der Fassung der Bekanntmachung vom 17. Juli 2013, BGBl. I S. 2538, das zuletzt durch Artikel 10 Absatz 3 des Gesetzes vom 27. Juli 2021, BGBl. I S. 3274, geändert worden ist) biologisch abbaubar. Beispiele für ein derartiges Tensid sind einfache, besonders bevorzugt flüssige, Schmierseifen (Kaliumseifen, Natriumseifen etc.) als anionisches Tensid.

Der Vorteil eines derart biologisch abbaubaren Tensides liegt insbesondere in der unproblematischen Entsorgung der Reinigungsflüssigkeit, da auch die in ihr enthaltenen Viren(-reste) gesundheitlich unbedenklich sind. Eine Entsorgung kann z.B. in den gewöhnlichen Haus-Abfluss (Stadtabwasser) erfolgen.

Nunmehr erfolgt eine Beschreibung einer erfindungsgemäßen Filterstufe.

Die Filterstufe ist für eine Vorrichtung zur Reduktion der Last behüllter Virionen in aerosolhaltiger Umgebungsluft vorgesehen. Die Vorrichtung umfasst ein Gehäuse und ein Gebläse, mittels dessen die Umgebungsluft durch das Gehäuse förderbar ist. Die Filterstufe umfasst eine Einrichtung zur Bereitstellung einer mit einer Reinigungsflüssigkeit benetzbaren Oberfläche, mit dem Ziel, eine Kontaktierung zwischen aerosolhaltiger Luft einerseits und Reinigungsflüssigkeit andererseits zu ermöglichen. Die Erfindung ist dadurch gekennzeichnet, dass die Reinigungsflüssigkeit tensidhaltig und vom fettlösenden Typ ist, so dass bei Kontakt die behüllte Virionen enthaltenden Aerosole mit der Oberfläche eine Inaktivierung dieser Virionen unter Beibehaltung der Integrität ihres proteinhaltigen Kerns erfolgt.

Eine derartige Filterstufe ist insbesondere zur Reduktion der Belastung der Umgebungsluft von behüllten Viren einsetzbar. Sie kann in geschlossenen Räumen eingesetzt werden, und weist eine hohe Energieeffizienz auf. Sie ist einfach und sehr kostengünstig zu warten, und es gehen weder Gesundheitsgefahren noch Umweltbeeinträchtigungen von ihr aus.

Zur Vermeidung von Wiederholungen wird auf die vorstehenden Erläuterungen zum erfindungsgemäßen Verfahren verwiesen.

Es sei angemerkt, dass das erfindungsgemäße Verfahren insbesondere mit der erfindungsgemäßen Filterstufe durchgeführt werden kann, und dass die erfindungsgemäße Filterstufe zur Durchführung des erfindungsgemäßen Verfahrens geeignet ist.

Nach einer bevorzugten Ausführungsform ist die Einrichtung zur Bereitstellung der mit der Reinigungsflüssigkeit benetzbaren Oberfläche eine Füllkörperkolonne, mittels der eine Grenzfläche des Tensidfilmes bereitstellbar ist.

Bei einer Füllkörperkolonne tritt die zu reinigende Umgebungsluft von unten in ihr Gehäuse ein und durchströmt von unten nach oben eine Füllkörperschüttung. Die Reinigungsflüssigkeit wird typischerweise von oben auf die Schüttung aufgegeben und durchläuft diese nach unten im Gegenstrom. Dabei erfolgt eine Benetzung der Füllkörperschüttung; an den Oberflächen der Füllkörper wird die erwünschte Grenzfläche bereitgestellt.

Nach einer weiteren Ausführungsform umfasst die Filterstufe einen Sammelbehälter zum Auffangen der die Einrichtung zur Bereitstellung der mit der Reinigungsflüssigkeit benetzbaren Oberfläche verlassenden, also der die Füllkörperkolonne herabrieselnden, Reinigungsflüssigkeit.

Aus dem Sammelbehälter ist die Reinigungsflüssigkeit, ggf. in Abhängigkeit des pH-Wertes, erneut auf die Füllkörperkolonne förderbar, oder als unbedenkliches Schmutzwasser entsorgbar. Es ist klar, dass das Volumen des Sammelbehälters ausreichend zu bemessen ist, und dass ein Zu- sowie Abfluss vorzusehen sind, um frische Reinigungsflüssigkeit oder frisches fettlösendes Tensid hinzufügen, oder verbrauchte Reinigungsflüssigkeit entsorgen zu können.

Da die Reinigungsflüssigkeit weder infektiös noch umweltbelastend ist, sind hier vorteilhafterweise keine besonderen Sicherheitsmaßnahmen erforderlich.

Die Erfindung betrifft auch eine Vorrichtung zur Reduktion der Last behüllter Virionen in aerosolhaltiger Umgebungsluft, umfassend eine Filterstufe, sowie ein Gehäuse und ein Gebläse gemäß obenstehender Definition. Anders ausgedrückt, die Erfindung ist auch auf ein Gerät ausgerichtet, welches die oben beschriebene Filterstufe umfasst, mittels welcher das erfindungsgemäße Verfahren durchführbar ist. Auf die vorstehenden Ausführungen wird daher verwiesen.

Nach einer Ausführungsform ist die Vorrichtung eine mobile raumluft-technische Einrichtung. Das bedeutet, dass dieselbe trag- oder fahrbar ausgestaltet ist, um an verschiedenen Einsatzorten aufgebaut und betrieben werden zu können.

Nach einer anderen Ausführungsform ist die Vorrichtung, oder zumindest die erfindungsgemäße Filterstufe, Teil einer stationären raumluft-technischen Einrichtung. Das bedeutet, dass die Vorrichtung oder Filterstufe fest in eine z.B. gebäudetechnische Anlage zur Luftaufbereitung oder Luftbehandlung integriert ist.

Die Erfindung betrifft auch die Verwendung einer Filterstufe oder Vorrichtung nach vorstehender Beschreibung zur Reduktion der Last behüllter Viren, wobei dies die Last der Umgebungsluft betrifft. Die Verwendung erfolgt unter Benutzung der vorstehend beschriebenen Filterstufe bzw. Vorrichtung, sowie unter Durchführung des vorstehend beschriebenen, erfindungsgemäßen Verfahrens. Dabei werden, im Unterschied zu bekannten Verfahren, die Virionen mittels Auflösung ihrer Hülle unter Beibehaltung der Integrität ihrer proteinhaltigen Kerne inaktiviert. Die lipidlösenden Bestandteile der Reinigungsflüssigkeit gelangen über eine Grenzfläche der Reinigungsflüssigkeit in die Aerosole, welche sich dann von der Grenzfläche ablösen.

Die Virionen werden in den Aerosolen durch die lipidlösenden Bestandteile der Reinigungsflüssigkeit ihrer Hülle beraubt und damit inaktiviert, und schließlich mit der übrigen Luft abtransportiert.

Die Verwendung einer Filterstufe oder Vorrichtung der vorstehend beschriebenen Art unter Durchführung des beschriebenen Verfahrens unterscheidet sich von den bekannten Verwendungen beispielsweise dadurch, dass diese eine Zerstörung der Viren durch chemische/physikalische Einwirkungen, eine Interaktion der Viren mit der Reinigungsflüssigkeit innerhalb derselben, einen Abtransport der Viren(reste) mittels der Flüssigkeit, und/oder eine Inaktivierung durch Zerstörung der proteinhaltigen Bestandteile der Viren betreffen.

Eine weitere Verbesserung wird erreicht, indem mittels der Vorrichtung trockene Luft aus der Umgebung eingesogen und befeuchtete Luft in die Umgebung abgegeben wird. Tatsächlich ist die Lebensfähigkeit von insbesondere behüllten Viren in trockener Luft, wie sie insbesondere zur kalten Jahreszeit, in der wiederum die Fenster eher geschlossen sind, höher als in feuchterer Luft. Die Vorrichtung kann also - unabhängig von der in ihrem Inneren ablaufenden Reinigung der sie tatsächlich durchströmenden Umgebungsluft - die Belastung der Umgebungsluft, die überhaupt nicht durch die Vorrichtung strömt, ebenfalls reduzieren, indem befeuchtete Luft in die Umgebung abgegeben wird, wo sie sich mit der "äußeren" Umgebungsluft vermischt und diese ebenfalls befeuchtet.

### Figurenbeschreibung

Nachfolgend wird die Erfindung anhand von Figuren beispielhaft erläutert. Dabei zeigt
- **Figur 1**: ein Flussdiagramm des erfindungsgemäßen Verfahrens;
- **Figur 2**: eine schematische Darstellung der Komponenten einer erfindungsgemäßen Vorrichtung.

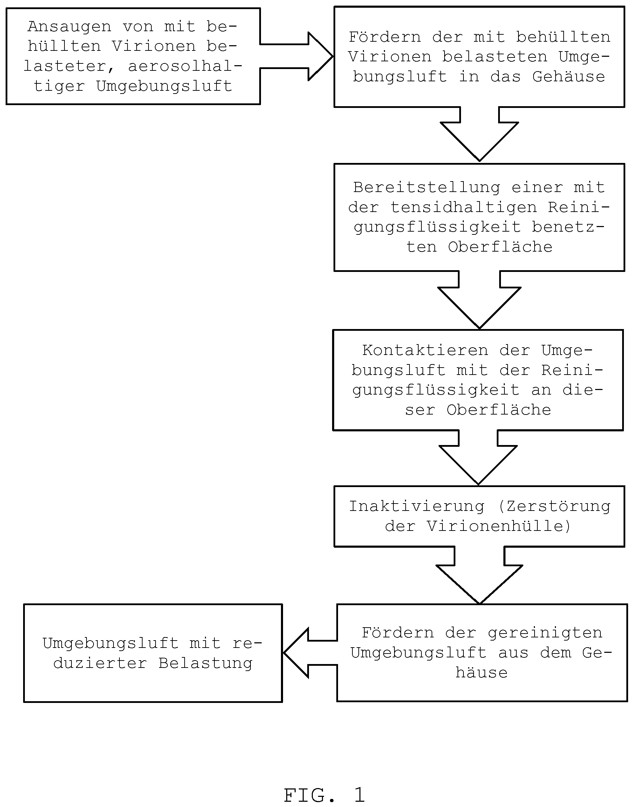

In der **Figur 1** ist ein vereinfachtes Flussdiagramm des erfindungsgemäßen Verfahrens dargestellt.

Demnach wird zunächst aus der Umgebung Luft, welche durch behüllte Viren belastete Aerosole enthält, angesogen und in ein Gehäuse gefördert. Dort ist eine mit einer tensidhaltigen, fettlösenden Reinigungsflüssigkeit benetzte Oberfläche bereitgestellt. Die an dieser Oberfläche entlanggeführte Umgebungsluft kontaktiert besagte Oberfläche; dort kommt es zu einer Interaktion der Aerosole und der in ihnen enthaltenen behüllten Viren mit der Reinigungsflüssigkeit. Aufgrund der Tensidhaltigkeit werden die Lipidhüllen der behüllten Viren aufgebrochen und die Viren so inaktiviert. Die proteinhaltigen Kerne hingegen bleiben intakt. Schließlich kann die so gereinigte, d.h. geringer mit (aktiven) behüllten Viren belastete Luft aus dem Gehäuse herausgefördert werden. Dort vermischt sie sich mit der verbleibenden Umgebungsluft und reduziert so auch die Belastung von Umgebungsluft, welche das Gehäuse nicht durchlaufen hat.

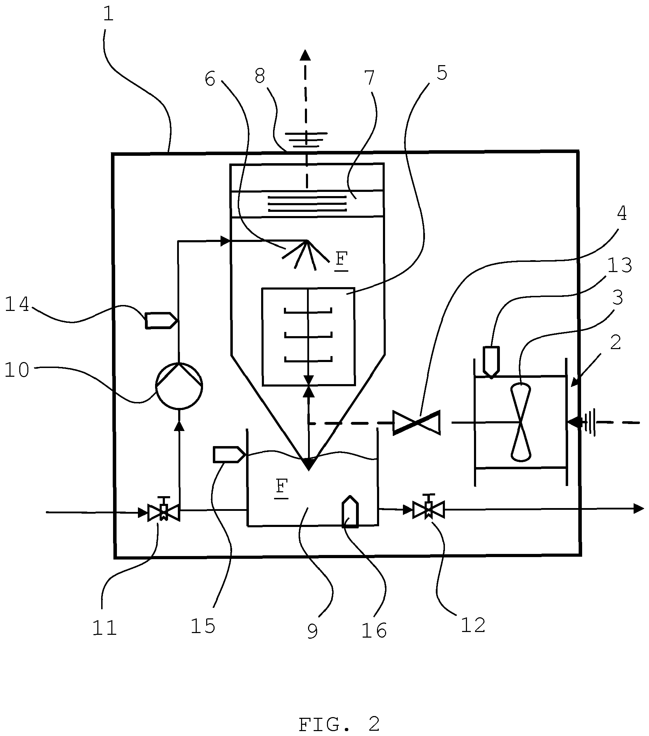

In der **Figur 2** ist eine schematische Darstellung der Komponenten einer bevorzugten Ausführungsform der erfindungsgemäßen Vorrichtung gezeigt.

An einem Gehäuse 1, welches stationär oder mobil sein kann, ist ein Ansauggitter mit Grobfilter 2 und Gebläse 3 angeordnet. Größere Partikel, Insekten oder dergleichen werden an dieser Stelle zurückgehalten, ebenso dient das Ansauggitter 2 dem Schutz vor Verletzungen. Über eine Drosselklappe 4, mittels welcher die Luftwechselrate an das Raumvolumen angepasst werden kann, gelangt die mit Aerosolen mit behüllten Viren angereicherte Umgebungsluft (gestrichelter Pfeil) in das Innere des Gehäuses 1. Ein Differenzdrucksensor 13 überwacht diesen Vorgang.

Die angesogene Umgebungsluft passiert nach einer Umlenkung eine Füllkörperkolonne 5, deren Oberflächen mit einer tensidhaltigen, fettlösenden Reinigungsflüssigkeit F benetzt sind. Das Benetzen erfolgt mittels einer Pumpe 10, die die Reinigungsflüssigkeit F von oben über eine Berieselungseinheit 6 auf die Füllkörperkolonne 5 bringt. Die Füllkörperkolonne 5 ist derart ausgelegt, dass der Luftstrom die Grenzfläche des Tensidfilmes passieren muss. Die sich durch die Länge der Füllkörperkolonne 5 ergebende Kontaktzeit stellt sicher, dass mittels des fettlösenden Tensids die in den Aerosolen enthaltenen Lipidhüllen der behüllten Viren aufgebrochen werden, wohingegen die proteinhaltigen Kerne intakt bleiben. Alle im Luftstrom enthaltenen Viren werden so inaktiviert, ohne dass eine vorhergehende Aktivierung der Reinigungsflüssigkeit F (z.B. mittels Erhitzens) nötig wäre, und ohne dass gesundheitsschädliche, desinfizierende Chemikalien, oder energieintensive Beleuchtungen benötigt werden.

Die nunmehr gereinigte Umgebungsluft durchströmt anschließend einen Tropfenabscheider 7 zur Verringerung von Mitreißeffekten von Tropfen. Schließlich verlässt die Umgebungsluft die Vorrichtung durch ein Ausblasgitter 8 als von aktiven behüllten Viren befreite Raumluft.

Im Sammelbehälter 9 befindet sich Reinigungsflüssigkeit F. Optional ist dieser durch ein Fenster einsehbar und mittels geeigneter Markierungen versehen (jeweils nicht dargestellt), so dass auch das Ansetzen der Reinigungsflüssigkeit F, beispielsweise aus lipidlösendem Biotensid und Wasser, auf einfache Weise möglich ist. Ein Füllstandssensor 15 überwacht die korrekte Füllstandsmenge. Das Wasser kann mittels eines Befüllventils 11 zugeführt bzw. aufgefüllt werden. Die korrekte Konzentration der Reinigungsflüssigkeit F wird mittels eines pH-Sensors 16 überwacht, die die Pumpe 10 freigibt.

Die Pumpe 10 fördert vorzugsweise erst nach Erreichen eines Mindestfüllstands im Sammelbehälter 9 ein vorwählbares Flüssigkeitsvolumen an das im Bild obenliegende Ende der Füllkörperkolonne 5. Die geförderte Flüssigkeitsmenge wird über einen Volumenstromsensor 14 überwacht. Nach dem schwerkraftgetriebenen Durchlaufen der Füllkörperkolonne 5 sammelt sich die Reinigungsflüssigkeit F wieder im Sammelbehälter 9. Dort kann optional anhand des pH-Sensors 16 die Lösungskonzentration permanent auf Wirksamkeit überwacht werden. Die Reinigungsflüssigkeit F zirkuliert dann erneut mittels Pumpe 10.

Vorzugsweise wird der Luftstrom erst nach einer eingestellten Vorlaufzeit der Pumpe 10 verzögert aktiviert. Hierdurch ist die Benetzung und somit das Herstellen der für den Betrieb der Vorrichtung benötigten Grenzflächen gewährleistet. Vorteilhaft ist ferner eine Positionierung des Ansauggitters 2 möglichst weit oben (oberhalb der Atemhöhe von ca. 1,6 - 1,8m), und des Ausblasgitters in Bodennähe, zum Erreichen einer Quellströmung, welche die natürliche Thermik im Raum unterstützt. Die Luftwechselzahl, also der Anteil der nach einer Stunde ausgetauschten bzw. gereinigten Luft, sollte bevorzugt ca. 4 bis 6 betragen (4 - 6-fache Umwälzung der gesamten Raumluft je Stunde). Dies entspricht einer Menge von z.B. 800 m³/h für ein Raumvolumen von 133 m³.

Ist die Reinigungsflüssigkeit F verbraucht, so kann sie mittels des Entleerventils 12 in das Hausabwasser entsorgt werden, sofern als fettlösendes Tensid ein biologisch abbaubares Tensid Verwendung findet.

Zur Vermeidung von Bakterienentwicklung in feuchtem Milieu kann das Gebläse 3 in einem Wartungsmodus nach vordefinierter Stillstandszeit auch ohne Inaktivierungsfunktion (d.h. mit stehender Pumpe 5) betrieben werden, so dass die Vorrichtung von Innen trocknet.

### Bezugszeichenliste

- 1: Gehäuse
- 2: Ansauggitter mit Grobfilter
- 3: Gebläse
- 4: Drosselklappe
- 5: Füllkörperkolonne
- 6: Berieselungseinheit
- 7: Tropfenabscheider
- 8: Ausblasgitter
- 9: Sammelbehälter
- 10: Pumpe
- 11: Befüllventil
- 12: Entleerventil
- 13: Differenzdrucksensor
- 14: Volumenstromsensor
- 15: Füllstandssensor
- 16: pH-Sensor
- F: Reinigungsflüssigkeit

## Patentansprüche

1. Verfahren zur Reduktion der Last behüllter Virionen in aerosolhaltiger Umgebungsluft, wobei die Umgebungsluft mittels eines Gebläses (3) durch ein Gehäuse (1) gefördert wird, in welchem sie mit einer Reinigungsflüssigkeit (F) in Kontakt gelangt, resultierend in einer Reduktion der in der Umgebungsluft befindlichen Last behüllter Virionen, wobei die Reduktion durch eine Inaktivierung der mit der Reinigungsflüssigkeit (F) in Kontakt gelangenden, in den Aerosolen enthaltenen behüllten Virionen erreicht wird, indem die Reinigungsflüssigkeit (F) tensidhaltig ist, **dadurch gekennzeichnet, dass** die Reduktion durch Interaktion in den Aerosolen mittels Gelangens des unbeeinflussten, fettlösenden Tensids aus einer Grenzfläche der Reinigungsflüssigkeit (F) in die Aerosole sowie einem Wiederablösen der Aerosole von der Reinigungsflüssigkeit (F), und der Abtransport der Virenreste mittels der Aerosole in die Umgebungsluft erfolgt, wobei lediglich die lipidhaltige Virushülle zerstört und das Virus somit unter Beibehaltung der Integrität seines proteinhaltigen Kerns inaktiviert wird.

2. Verfahren nach Anspruch 1, wobei die zu reinigende Umgebungsluft an einer einen Tensidfilm bereitstellenden Einrichtung zur Bereitstellung einer mit einer Reinigungsflüssigkeit (F) benetzten Oberfläche entlang geführt wird, welche derart ausgelegt ist, dass die Umgebungsluft eine Grenzfläche des Tensidfilmes passieren muss, wobei die Kontaktzeit zwischen Aerosolen und Grenzfläche dergestalt ausgewählt ist, dass das Tensid die in den Aerosolen enthaltenen Lipidhüllen der behüllten Virionen aufbricht, wodurch diese Virionen inaktiviert werden.

3. Verfahren nach einem der vorhergehenden Ansprüche, wobei die Umgebungsluft im Wege des Kontaktierens der Reinigungsflüssigkeit (F) befeuchtet wird, um die Lebensdauer von Virionen in der Umgebungsluft zu reduzieren.

4. Verfahren nach einem der vorhergehenden Ansprüche, wobei die aus dem Gehäuse (1) herausgeförderte, gereinigte Umgebungsluft in einen Innenraum zurückgefördert wird.

5. Verfahren nach einem der Ansprüche 2 bis 4, wobei die Reinigungsflüssigkeit (F) mittels Schwerkraft entlang der Einrichtung zur Bereitstellung einer mit einer Reinigungsflüssigkeit (F) benetzbaren Oberfläche fließt und sich in einem Sammelbehälter (9) sammelt, von wo aus sie in Abhängigkeit des pH-Wertes erneut auf die Einrichtung zur Bereitstellung einer mit einer Reinigungsflüssigkeit (F) benetzbaren Oberfläche gegeben, oder als unbedenkliches Schmutzwasser entsorgt wird.

6. Verfahren nach einem der vorhergehenden Ansprüche, wobei das Tensid gemäß Verordnung (EG) Nr. 648/2004 biologisch abbaubar ist.

7. Filterstufe für eine Vorrichtung zur Reduktion der Last behüllter Virionen in aerosolhaltiger Umgebungsluft, die Vorrichtung umfassend ein Gehäuse (1) und ein Gebläse (3), mittels dessen die Umgebungsluft durch das Gehäuse (1) förderbar ist, wobei die Filterstufe eine Einrichtung zur Bereitstellung einer mit einer Reinigungsflüssigkeit (F) benetzbaren Oberfläche umfasst, **dadurch gekennzeichnet, dass** die Reinigungsflüssigkeit (F) tensidhaltig und vom fettlösenden Typ ist, so dass bei Kontakt der behüllte Virionen enthaltenden Aerosole mit der Oberfläche eine Inaktivierung dieser Virionen unter Beibehaltung der Integrität ihres proteinhaltigen Kerns erfolgt.

8. Filterstufe nach Anspruch 7, wobei die Einrichtung zur Bereitstellung der mit der Reinigungsflüssigkeit (F) benetzbaren Oberfläche eine Füllkörperkolonne (5) ist, mittels der eine Grenzfläche des Tensidfilmes bereitstellbar ist.

9. Filterstufe nach einem der Ansprüche 7 oder 8, ferner umfassend einen Sammelbehälter (9) zum Auffangen der die Einrichtung zur Bereitstellung der mit der Reinigungsflüssigkeit (F) benetzbaren Oberfläche verlassenden Reinigungsflüssigkeit (F), aus welchem diese in Abhängigkeit des pH-Wertes erneut auf die Füllkörperkolonne (5) förderbar, oder als unbedenkliches Schmutzwasser entsorgbar ist.

10. Vorrichtung zur Reduktion der Last behüllter Virionen in aerosolhaltiger Umgebungsluft, umfassend eine Filterstufe, sowie ein Gehäuse (1) und ein Gebläse (3) gemäß Definition in Anspruch 7.

11. Vorrichtung nach Anspruch 10, wobei dieselbe eine mobile raumluft-technische Einrichtung ist.

12. Vorrichtung nach Anspruch 10, wobei dieselbe Teil einer eine stationären raumluft-technischen Einrichtung ist.

13. Verwendung einer Filterstufe nach einem der Ansprüche 7 bis 9, oder einer Vorrichtung nach einem der Ansprüche 10 bis 12, jeweils unter Nutzung eines Verfahrens nach einem der Ansprüche 1 bis 6, zur Reduktion der Last behüllter Virionen in der Umgebungsluft, indem die Virionen mittels Auflösung ihrer Hülle unter Beibehaltung der Integrität ihrer proteinhaltigen Kerne inaktiviert werden.
